# EUROPEAN PATENT APPLICATION

(11) **EP 2 845 536 A1**
(43) Date of publication of application: **11.03.2015**
(21) Application number: 14183625.4
(22) Date of filing: 04.09.2014
(51) Int. Cl.: A61B 5/00, A61B 5/01, G08B 21/04, H04N 7/18

(54) **Infant monitoring apparatus**

(30) Priority: 04.09.2013 GB 201315696
(71) Applicant: Binatone Electronics International Ltd, Sheung Wan, Hong Kong (CN)
(72) Inventor: Lee, Heung Sang, Sheung Wan, Hong Kong (CN); Chow, Hung Pong, Sheung Wan, Hong Kong (CN)
(74) Representative: Baldwin, Mark

(57) **Abstract**

A portable receiver (12) of an infant monitoring apparatus (10) that includes the receiver and an infant monitoring device (14) configured to transmit infant monitoring data from a first location at which an infant is located to a second location, remote from the first location, at which the portable receiver is located. The portable receiver (12) has receiver to receive infant monitoring data from the infant monitoring device, a non-contact temperature sensing device by which an infant's temperature can be sensed without touching the infant and a display to display temperatures sensed by the non-contact temperature sensing device

## Description

### Field of the Invention

The invention relates infant monitoring apparatus.

### Background to the Invention

Infants, including babies, are often put to bed in one location while parents, or other carers, relax in another. In these circumstances it is desirable for the parent or carer to be able to monitor the condition of the infant without having to go the infant's bedside.

### Summary of the Invention

The invention provides an infant monitoring apparatus as specified in claim 1.

The invention also includes a method of operating a portable receiver of an infant monitoring apparatus as specified in claim 13.

### Brief Description of the Drawings

In the disclosure that follows, reference will be made to the drawings in which:
Figure 1 is shows an infant monitoring apparatus comprising a portable receiver and an infant monitoring device;
Figure 2 is a schematic illustration of the infant monitoring device;
Figure 3 is a schematic illustration of the portable receiver;
Figure 4 is a rear elevation of the portable receiver; and
Figure 5 shows the portable receiver in use.

### Detailed Description

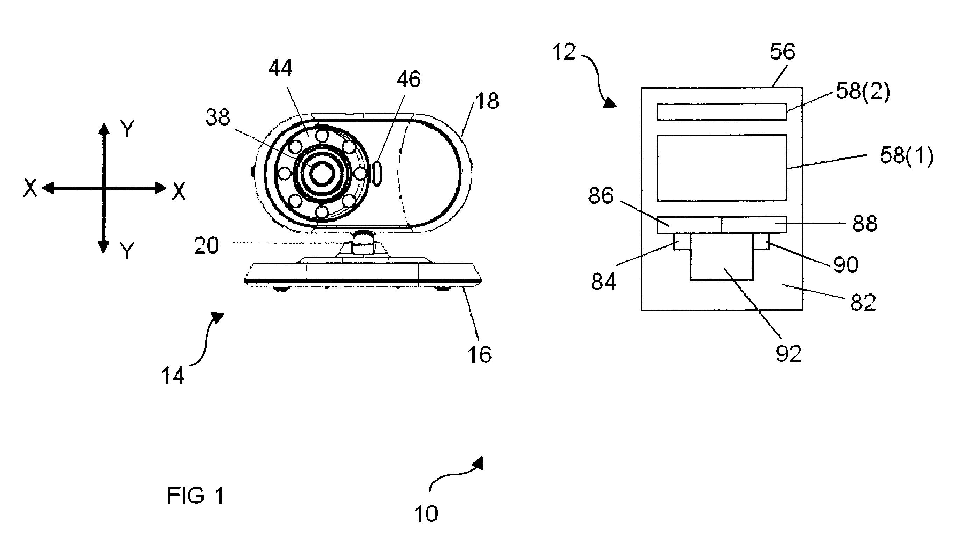

Figure 1 shows an infant monitoring apparatus 10 comprising a portable receiver 12 and an infant monitoring device 14. The infant monitoring device 14 is to be located at a first location, such as a bedroom, at which a infant has been placed, typically to sleep. The infant monitoring device 14 is configured to transmit infant monitoring data to the portable receiver 12. The portable receiver 12 may be held by a user, such as a parent or other carer, at a second location remote from the first location. The first and second locations may be on different floors of a building, or the first may be within a building while the second is without.

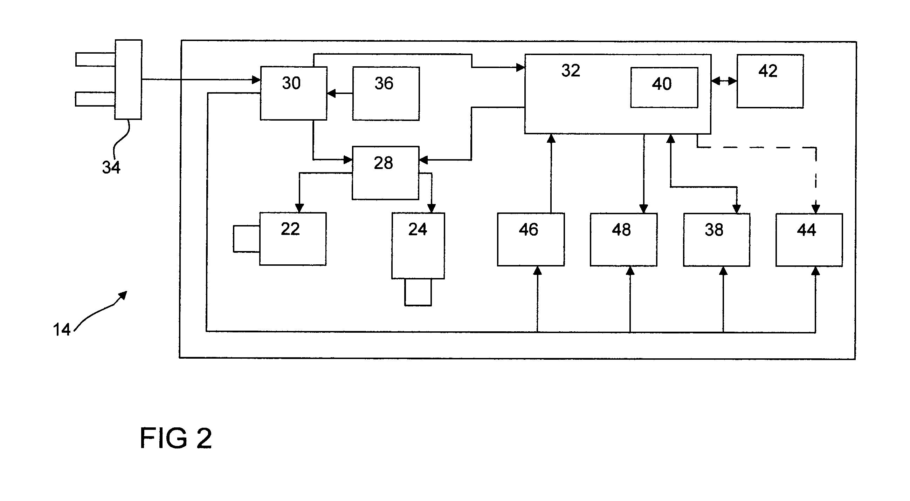

Referring to Figures 1 and 2, the infant monitoring device 14 comprises a base 16 and a housing 18 mounted on the base. The connection 20 between the housing 18 and the base 16 comprises an articulation so that the housing can be moved relative to the base by a drive unit mounted in the housing. The drive unit comprises two motors 22, 24 that can be powered to cause the housing to pivot about the X and Y axes. The motors 22, 24 may be stepper motors. The drive unit further comprises a motor controller 28 to control actuation of the motors 22, 24. The motor controller 28 may receive electric power from a power supply unit 30 and actuate the motors 22, 24 in response to command signals issued by a device controller 32 in response to command signals received from the portable receiver 12. The power supply unit 30 may distribute electric power received from a mains supply via an ac/dc adapter provided in a plug 34 by which the infant monitoring device 14 can be connected to a mains electric supply. In other examples, the ad/dc adapter may be a part of the power supply unit 30. In some examples, the power supply unit 30 may also distribute electrical power received from a battery supply 36 housed in a compartment provided in the infant monitoring device 14.

The infant monitoring device 14 comprises an image capturing device 38. The image capturing device 38 comprises a video camera having one or more lenses and a semi-conductor device that receives light via the lens(es) and records the received light electronically. The semi-conductor device may be a 1/6.5" VGA CMOS image sensor. The image capturing device 38 is connected with the device controller 32. The device controller 32 comprises a processor 40 that is operable to process digital video data output by the image capturing device 38 into digital video frame data for transmission to the portable receiver 12. The processor 40 may be provided with an integral buffer memory to buffer the digital video frame data. Alternatively, a separate buffer memory may be provided. The buffer memory may comprise volatile RAM provided by an SRAM or DRAM module. The device controller 32 is connected with a signal transmitter/receiver 42 that is operable to convert the digital video frame data into a format suitable for wireless transmission to the portable receiver 12. In the illustrated example, the format used is a frequency hopping spread spectrum (FHSS) format. The transmission may be in the 2.4GHz waveband.

The infant monitoring device 14 may further comprise a night vision unit 44. The night vision unit 44 may comprise a plurality of LEDs activated automatically by a light sensor. In some examples, the night vision unit 44 may be actuated by a clock signal from the device controller 32 or a signal issued by the device controller in response to a command signal issued by a user using a suitably equipped portable receiver 12 (these optional or alternative modes are indicated in Figure 2 by a dashed line connection between the device controller 32 and night vision unit 44). In the illustrated example, the night vision unit 44 comprises an array of eight LEDs disposed at equi-spaced intervals on a pitch circle diameter so as to surround the image capturing device 38.

The infant monitoring device 14 may further comprise an audio capturing device, such as a microphone 46. The microphone 46 may be incorporated in the image capturing device 38. In example in which a microphone is included in the image capturing device 38, the image capturing device may be provided with an audio codec to encode analogue audio data as digital data for transmission to the portable receiver 12 with the digital video data.

The digital video frame data and, where obtained, digital audio data transmitted by the infant monitoring unit 14 comprises infant monitoring data that is received by the portable receiver 12 and output by the portable receiver 12 in a form a user can access so as to be able to monitor an infant's condition.

The infant monitoring device 14 may further comprise an audio output device 48, such as a loudspeaker housed by the housing 18. In examples provided with an audio output device 48, the device controller 32 may be provided with an audio codec and output interface so as to be able to output an analogue signal to the audio output device. The audio output device 48, when provided, can be used to output audio received from portable receivers 12 configured to transmit audio that is input contemporaneously by a user, or output soothing messages or other sounds stored in non-volatile memory accessible by the device controller 32.

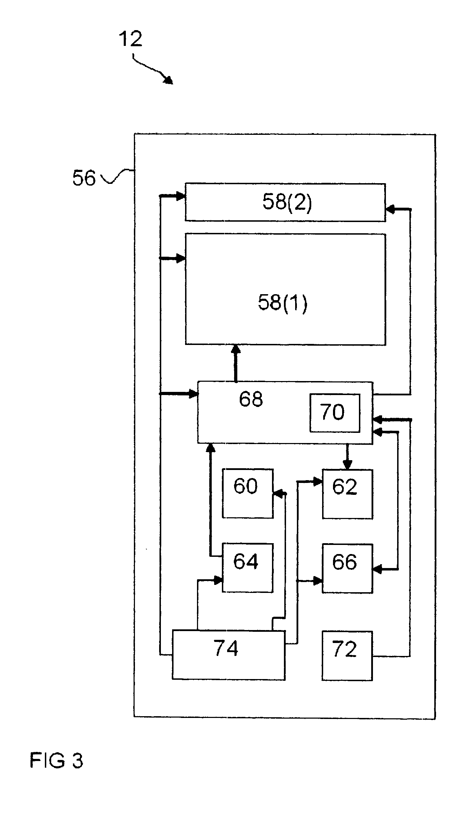

Referring to Figures 1 and 3, the portable receiver 12 comprises a handheld housing 56, a display 58(1), 58(2) and a non-contact temperature sensing device 60 housed by the housing. The portable receiver 12 may further comprise an audio output device 62, an audio capturing device 64, a transmitter/receiver 66, a device controller 68 that includes a processor 70, an input interface 72 and a power supply 74.

The display may comprise a TFT LCD screen 58(1) to display video images derived from infant monitoring data received from the infant monitoring unit 14. The display may additionally comprise an audio level indicator 58(2). The audio level indicator 58(2) may comprise an array of LEDs to provide a visual sound level indication for the audio output by the audio output device 62 or the audio signal captured by the audio capturing device 64. The array of LEDs may comprise a series of LEDs arranged in line.

The transmitter/receiver 66 is configured to receive infant monitoring data transmitted by the infant monitoring device 14 and communicate it to the device controller 68 in a form the processor 70 can process for display via the screen 58(1). In examples in which the infant monitoring device 14 is configured to transmit digital audio data to the portable receiver 12, the device controller 68 is provided with an audio codec and output interface for outputting an analogue signal to the audio output device 62. The audio output device 62 may be a loudspeaker housed by the housing 56. Additionally, or alternatively, the audio output device 62 may be an output jack that allows the portable receiver 12 to be connected to an earphone or external speaker.

The audio capturing device 64 may comprise a microphone housed by the housing 56. The audio capturing unit 64 may be connected with the device controller 68 to enable analogue audio data received from the microphone to be encoded as digital data for transmission by the receiver/transmitter 66 to the infant monitoring device 14. Providing the portable receiver 12 with this facility allows a user, to send soothing messages to the infant via an audio output device of the infant monitoring device 14.

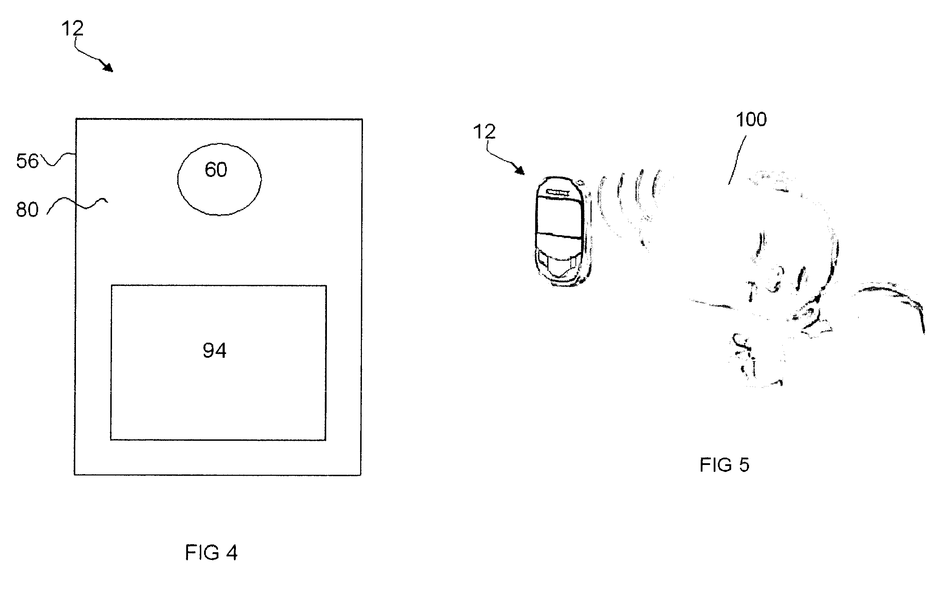

Referring to Figure 4, the non-contact temperature sensing device 60 may be housed by the housing 56 so as to point from a major face 80 of the housing disposed opposite the major face 82 (Figure 1) at which the display 58 is visible. The temperature sensing device 60 may be an infra red thermometer configured to output a digital signal indicative of a sensed temperature. The digital signals output the by the temperature sensing device 60 are communicated to the processor 70, which is configured to process signals from the temperature sensing device 60 and output a signal to the display that causes a temperature reading to be displayed on the screen 58(1). In other examples, the portable receiver 12 may be provided with a further display device to display the temperature reading. However, it is convenient and economic to display the temperature reading using the screen 58(1).

Referring to Figure 1, the interface unit 72 of the portable receiver 12 may comprise a plurality of buttons to allow a user, to input commands to the portable receiver. In the illustrated example, the interface unit 72 comprises:
a power on/off button 84;
a video on/off button 86;
a temperature sensor on/off button 88;
an audio input device on/off and recording level adjustment button 90; and
a multi-function pad 92 operable to provide volume +/- and pan/tilt and zoom control inputs for the image capturing device 38.

The power supply 74 distributes electrical power received from a battery supply housed in a compartment housed by the housing 56 and accessible via a cover 94 (Figure 4) provided in the major face 80. The battery supply may be rechargeable by removal from the housing 56 and connection with a suitable battery charger. Alternatively, the portable receiver 12 may be provided with an input socket (not shown) to allow the battery supply to be connected to a charger unit that may be a part of a plug (not shown) that can be plugged into a mains electrical supply or allow the portable receiver to be plugged onto a charger cradle (not shown). In another example the battery supply may be recharged by an inductive charging system.

In use, a user can situate the infant monitoring device 14 in a room or area in which an infant is to be left, typically to sleep and go to another room or area with the portable receiver 12. The portable receiver 12 can receive infant monitoring data transmitted by the infant monitoring device 14 allowing the user to view the infant via the screen 58(1) and hear sounds via the audio output device 62. By operation of the multi-function pad 92 the user can cause command signals to be transmitted to the infant monitoring device 14 to cause the image capturing device 38 to pan (pivot about the Y-Y axis), tilt (pivot about the X-X axis) or zoom in and out. The user can operate the button 90 to activate the audio capturing device 64 and send audio messages to the infant via the audio output device 48 of the infant monitoring device. Additionally, the user can take the portable receiver 12 to the infant and operate the button 88 to activate the non-contact temperature sensing device 60 and obtain a reading of the infant's temperature, which will be displayed on the screen 58(1). This has the advantage that the temperature reading can be obtained without disturbing the infant and even in cases in which the infant is awake, avoids having to obtain the infant's cooperation in using a contact temperature sensor such as a mercury thermometer. It s to be understood that while the temperature sensing device 60 is intended primarily for obtaining an infant's temperature, it can be used to obtain non-contact temperature readings of other things.

## Claims

1. An infant monitoring apparatus comprising a portable receiver (12) and an infant monitoring device (14) having a transmitter (42) configured to transmit infant monitoring data wirelessly from a first location at which said infant is located to a second location at which said portable receiver is located, said portable receiver comprising a receiver (66) to receive said infant monitoring data, a non-contact temperature sensing device (60) by which an infant's temperature can be sensed without touching said infant and a display (58(1), 58(2)) to display temperatures sensed by said non-contact temperature sensing device.

2. An infant monitoring apparatus as claimed in claim 1, wherein said non-contact temperature sensing device comprises an infra red thermometer (60).

3. An infant monitoring apparatus as claimed in claim 1 or 2, wherein said temperature sensing device is a digital temperature sensing device (60).

4. An infant monitoring apparatus as claimed in claim 1, 2 or 3, wherein said portable receiver (12) further comprises an audio output device (62) to output audio derived from said infant monitoring data received from said infant monitoring device (14) and said infant monitoring device comprises an audio capturing device (46) to capture audio to be included in said infant monitoring data.

5. An infant monitoring apparatus as claimed in any one of the preceding claims, wherein said display (58(1)) is configured to display images derived from said infant monitoring data received from said infant monitoring device (14) and said infant monitoring device comprises an image capturing device (38) to capture said images.

6. An infant monitoring apparatus as claimed in claim 5, wherein said image capturing device comprises a video camera (38).

7. An infant monitoring apparatus as claimed in claimed in claim 5 or 6, wherein said infant monitoring device (14) comprises a drive unit (22 24) configured to move said image capturing device (38), said portable receiver (12) has an input device (72) configured to receive movement commands input by a user, said receiver (66) is a receiver/transmitter configured to transmit command signals based on said movement commands to said infant monitoring device and said drive unit operates to move said image capturing device in response to said command signals.

8. An infant monitoring apparatus as claimed in claim 7, wherein said input device (72) is configured to receive zoom commands input by a user and said receiver/transmitter (66) is configured to transmit command signals based on said zoom signals to said infant monitoring device (14) and said image capturing device (38) is configured to zoom in response to said zoom signals.

9. An infant monitoring device as claimed in any one of claims 5 to 8, wherein said infant monitoring device further comprises a night vision unit (44) to enable said image capturing device (38) to capture said images in low light conditions.

10. An infant monitoring apparatus as claimed in any one of the preceding claims, wherein said portable receiver (12) further comprises an audio capturing device (64), said receiver is a receiver/transmitter (66) configured to transmit audio data to said infant monitoring device (14) and said infant monitoring device comprises an audio output device (48) to output audio signals derived from said audio data.

11. An infant monitoring apparatus as claimed in any one of the preceding claims, wherein said transmitter (42) of said infant monitoring device (14) is configured to transmit said infant monitoring data using a frequency hopping spread spectrum (FHSS) format and said receiver (66) of said portable receiver (12) is configured to receive FHSS transmissions from said infant monitoring device.

12. An infant monitoring apparatus as claimed in any one of the preceding claims, wherein said portable receiver (12) further comprises a device controller (68) comprising a processor (70) configured to control operation of said portable receiver.

13. A method of operating a portable receiver of an infant monitoring apparatus comprising a portable receiver (12) and an infant monitoring device (14) configured to wirelessly transmit infant monitoring data to said portable receiver, said method comprising pointing said portable receiver at an infant to direct a non-contact temperature sensing device (60) of said portable receiver at said infant to obtain a temperature reading for said infant from said temperature sensing device.

14. A method of operating a portable receiver of an infant monitoring apparatus as claimed in claim 13, further comprising receiving infant monitoring data from said infant monitoring device using a frequency hopping spread spectrum transmission format.

15. A method of operating a portable receiver of an infant monitoring apparatus as claimed in claim 13 or 14, further comprising operating a user interface (72) to cause the transmission of commands to said infant monitoring device (14) to control operation of an image capturing device (38) of said infant monitoring device.
